# FASCICULE DE BREVET EUROPEEN

(11) **EP 3 247 273 B1**
(45) Date de publication et mention de la délivrance du brevet: **24.05.2023**
(21) Numéro de dépôt: 15830802.3
(22) Date de dépôt: 17.12.2015
(51) Int. Cl.: A61B 5/22, A61B 5/00, A61B 5/11

(54) **ERGOMETRE POUR CHEVILLE**
FUSSKNÖCHELERGOMETER
ANKLE ERGOMETER

(30) Priorité: 20.01.2015 FR 1550439
(43) Date de publication de la demande: 29.11.2017
(73) Titulaire: Université de Valenciennes et du Hainaut-Cambresis, 59313 Valenciennes Cedex 9 (FR); Centre National de la Recherche Scientifique (C.N.R.S.), 75016 Paris (FR)
(72) Inventeur: SIMONEAU-BUESSINGER, Emilie, 59121 Prouvy (FR); GILLET, Christophe, 59440 Semousies (FR); LETENEUR, Sébastien, 59171 Erre (FR); DEBRIL, Jean-François, 86000 Poitiers (FR); DECOUFOUR, Nicolas, 62232 Fouquieres les Bethune (FR)
(74) Mandataire: Plasseraud IP
(86) Numéro de dépôt international: PCT/FR2015/053578
(87) Numéro de publication internationale: WO 2016/116673

(56) Documents cités:
- US-A1- 2012 010 534
- US-A1- 2013 303 947

## Description

### Domaine technique et Art antérieur

La présente invention concerne le domaine des ergomètres, et plus particulièrement celui des ergomètres de type cheville.

Un des objets de la présente invention est de fournir un appareil de mesure apte à mesurer avec précision les propriétés mécaniques des muscles de la cheville, et notamment la capacité des muscles intervenant dans la mobilité de la cheville, et plus particulièrement la capacité des muscles de la cheville à exercer une force pour réaliser une flexion plantaire (ou extension) et/ou une flexion dorsale (ou dorsiflexion).

La présente invention trouve de nombreuses applications avantageuses dans le domaine médical et/ou paramédical ou encore dans le domaine sportif lors par exemple d'une préparation physique.

On peut citer comme exemple d'applications de la présente invention la réalisation de tests sur une personne afin d'évaluer sa capacité à maintenir une posture orthostatique (position debout) ; ceci est par exemple particulièrement avantageux dans un contexte médical pour décider d'autoriser un patient alité pendant une période prolongée à se mettre debout et reprendre une activité physique en toute sécurité.

D'autres applications avantageuses peuvent également être envisagées dans le cadre de la présente invention, comme par exemple pour réaliser des exercices de rééducation adaptés pour la personne suite par exemple à une entorse de la cheville, ou encore pour évaluer les progrès réalisés par une personne suite à une entorse de la cheville.

On sait que le système neuromusculaire a la capacité de modifier ses caractéristiques et/ou son comportement sous l'influence de facteurs endogènes et/ou exogènes tels que par exemple le vieillissement, l'hypoactivité ou encore l'hyperactivité.

Ce phénomène est connu sous le terme de plasticité neuromusculaire.

Ainsi, la capacité d'un muscle à produire une force peut augmenter ou diminuer en fonction de sa sollicitation.

Pour évaluer cette plasticité neuromusculaire, il existe des instruments de mesure de type ergomètres qui permettent d'évaluer l'impact du vieillissement, d'une immobilisation prolongée ou encore d'un entraînement ; ceci est réalisé sur la base d'une analyse des moments de force développés aux articulations.

Si l'on s'intéresse plus particulièrement à l'articulation de la cheville, on constate que la production de force sur l'articulation de la cheville pour une extension et une dorsiflexion du pied est particulièrement importante, notamment pour maintenir un individu en équilibre dans une posture orthostatique.

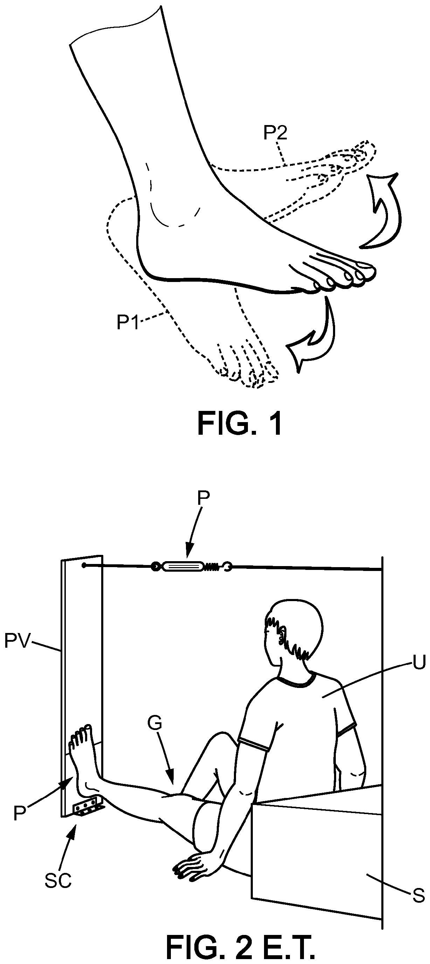

La figure 1 annexée à la présente description représente schématiquement un pied en flexion plantaire P1 et en dorsiflexion P2.

Ces deux principaux mouvements à la cheville (extension/dorsiflexion) sont effectués dans un plan sagittal autour d'un axe transversal à la cheville.

La dorsiflexion P2 permet de rapprocher le dos du pied de la face antérieure de la jambe ; l'extension P1 éloigne le dos du pied de la face antérieure de la jambe, amenant le pied à se placer dans le prolongement de la jambe.

Ainsi, en permettant une extension P1 et une dorsiflexion P2 actives, les muscles autour de l'articulation de la cheville permettent de maintenir un individu en équilibre dans une posture orthostatique et permettent également la locomotion (marche, course et déplacements latéraux).

On comprend donc qu'il est très important, notamment d'un point de vue médical, de pouvoir évaluer avec précision les propriétés mécaniques et fonctionnelles des muscles de la cheville, et notamment les forces (ou moments de force) produites par les muscles de la cheville en extension P1 et en dorsiflexion P2.

Ceci permet notamment d'évaluer la capacité d'un individu à produire une force musculaire suffisante pour se maintenir dans une posture orthostatique et/ou à reprendre une activité physique.

Classiquement, les appareils de mesure de type ergomètres comportent une pédale reliée :
- soit à un capteur de force unidirectionnel placé à distance de l'axe de rotation de la pédale,
- soit à un capteur de couple placé au niveau de l'axe de rotation de la pédale (c'est-à-dire aligné avec l'axe de rotation flexion - extension de l'articulation de la cheville).

Ces capteurs sont ainsi configurés pour mesurer la force ou le moment de force produit dans des conditions statiques.

Cette mesure se fait directement avec un capteur de couple ou indirectement avec un capteur de force.

Il faut en effet, pour le capteur de force, multiplier la force mesurée par le bras de levier entre l'axe de la pédale (axe cheville) et l'axe du capteur de force (point d'application de la force).

Sur la base de cette technique de mesure, il existe dans l'état de la technique plusieurs types d'ergomètres.

Un premier type d'ergomètre recensé dans l'état de la technique est représenté en figure 2.

Sur cette figure 2, on distingue un support S placé derrière le dos et le bassin de l'utilisateur U de sorte que le genou G de l'utilisateur soit en extension complète et que la plante du pied P soit en contact avec une planche verticale PV.

Cette planche PV est articulée au sol par un système de charnière SC permettant un pivotement de la planche par rapport au sol.

Un dynamomètre D est par ailleurs fixé entre cette planche verticale PV et le support S pour maintenir en position ladite planche dans un axe sensiblement vertical lorsqu'aucune force n'est exercée sur la planche par l'utilisateur.

Ce dynamomètre D est configuré en outre pour mesurer la force exercée au niveau de la plante du pied P suite à une flexion plantaire.

Un tel système est décrit dans "Absolute muscle force in the ankle flexors of man. " J. Physiol. (I944) I03, 267-273, Haxton, H. A. (1944).

Un tel système ne permet de mesurer que les forces exercées lors d'une flexion plantaire (ou extension), et ne permet pas de mesurer les forces exercées lors d'une flexion dorsale (ou dorsiflexion).

Il existe d'autres types d'ergomètres.

On connaît par exemple l'ergomètre développé par l'Institut Universitaire Technologique de Génie Mécanique de Dijon qui permet de mesurer les moments musculaires à l'articulation de la cheville via une pédale avec jauges de contraintes.

Avec un tel ergomètre, l'utilisateur doit être en position assise avec une hanche fléchie sensiblement à 110°, un genou fléchi sensiblement à 120°, et une cheville à 90° (voir notamment Simoneau et al. 2008: "Difficult memory task during postural tasks of varions difficulties in young and older people: a pilot study. " Clin Neurophysiol 119(5): 1158-65*, ou* Simoneau et al. 2009: "Antagonist mechanical contribution to resultant maximal torque at the ankle joint in young and older men. " J Electromyogr Kinesiol 19(2): 123-31*,* ou encore Billot et al. 2010: "Age-related relative increases in electromyography activity and torque according to the maximal capacity during upright standing. " Appl Physiol 109(4): 669-80*).*

On connaît encore l'ergomètre de type cheville développé par la société Bio2M.

Cet ergomètre est spécialement conçu pour tester les propriétés mécaniques des muscles fléchisseurs plantaires à l'articulation de la cheville (voir notamment Tognella et al. 1997 : "A mechanical device for studying mechanical properties of human muscles in vivo. " J Biomech 30(10): 1077-80*, ou* Ochala et al. 2004: "Changes in mechanical properties of human plantar flexor muscles in ageing." Exp Gerontol 39(3): 349-58*),* et notamment pour mesurer les propriétés contractiles des muscles suite par exemple à des contractions isométriques, isocinétiques et isotoniques.

Il est composé de deux unités principales:
(i) une unité de puissance qui contient notamment un vérin et un capteur de couple qui sont électroniquement reliés entre eux, et
(ii) une unité de commande contrôlée par un ordinateur.

Selon cet ergomètre, les déplacements angulaires de la cheville sont mesurés avec un capteur optique numérique, et les vitesses angulaires sont prises à partir d'un résolveur lié au rotor, sauf pour les vitesses supérieures à 15,70 *rad.s⁻¹* qui nécessitent un tachymètre.

Les couples sont obtenus en utilisant un capteur de couple à jauges.

L'utilisateur d'un tel ergomètre doit se tenir en position allongée sur le dos avec le genou fléchi à 120°.

Un tel ergomètre minimise la contribution des muscles gastrocnémiens (voir notamment Cresswell et al. 1995 : "Influence of gastrocnemius muscle length on triceps surae torque development and electromyographic activity in man." Exp Brain Res 105(2): 283-90*)* ; de tels muscles ont leur importance dans la flexion plantaire et leur contribution ne doit pas être minimisée et écartée de la mesure si l'on veut une mesure précise.

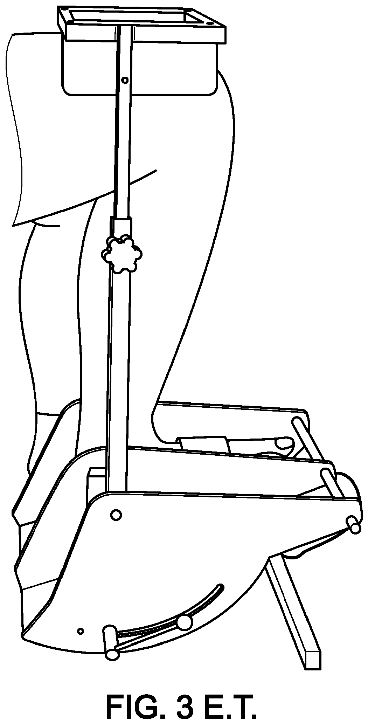

On connaît également un ergomètre tel que celui illustré en figure 3.

Avec un tel ergomètre, les mesures de la force isométrique en flexion plantaire sont effectuées avec une fixation stable du membre inférieur par blocage de l'articulation du genou à 90°, minimisant ainsi la rotation de l'articulation de la cheville lors de la contraction isométrique (voir notamment Reeves et al. 2005 : "Influence of 90-day simulated microgravity on human tendon mechanical properties and the effect of resistive countermeasures." J Appl Physiol 98(6): 2278-86*).*

Un dispositif de mesure de la spasticité d'une cheville d'un sujet est connue du document US 2013/0303947 A1 et permet de mesurer la force exercée sur l'articulation de la cheville.

Enfin, il existe plusieurs études menées sur l'articulation de la cheville qui utilisent le « *CybexNorm*^{®} » pour évaluer les moments appliqués par les muscles de la cheville (voir notamment Scaglioni et al. 2002: "Plantar flexor activation capacity and H reflex in older adults: adaptations to strength training. " J Appl Physiol 92(6): 2292-302*.,* ou Ferri et al. 2003: "Strength and power changes of the human plantar flexors and knee extensors in response to resistance training in old age. " Acta Physiol Scand 177(1): 69-78*).*

Le Demandeur soumet toutefois que l'ensemble des ergomètres de type cheville existants présente de nombreuses limites et de nombreux inconvénients.

En effet, sur la plupart des ergomètres, l'utilisateur peut prendre appui sur son dos ou sur ses épaules pour pousser plus fort sur la pédale.

Ceci implique nécessairement la mobilisation de muscles d'autres articulations, ce qui entraîne une surestimation de l'évaluation des forces ou des moments de force développés à la cheville et peut également entraîner une forte variabilité des mesures.

En d'autres termes, avec les ergomètres actuels, il est possible d'enregistrer un moment de force en flexion plantaire, sans utiliser les muscles de la cheville responsables de la flexion plantaire.

Ceci est dû au fait qu'avec les ergomètres actuels l'utilisateur peut solliciter d'autres muscles pendant l'effort pour réaliser une flexion plantaire, par exemple les muscles extenseurs de genou ou de hanche : la configuration des ergomètres actuels implique donc une erreur difficilement décelable qu'il est impossible de corriger *a posteriori.*

Les ergomètres de type cheville qui nécessitent un positionnement du genou fléchi à 90° essaient de limiter la participation de ces muscles extenseurs de genou ou de hanche.

Cependant, ces ergomètres réduisent également la sollicitation des muscles gastrocnémiens lors de la flexion plantaire, comme c'est le cas par exemple de l'ergomètre représenté en figure 3.

Or, comme mentionné ci-dessus, la capacité de production de force de ces muscles gastrocnémiens est importante pour l'évaluation de la flexion plantaire en position jambe tendue (en extension) : en effet, ces muscles gastrocnémiens doivent nécessairement être pris en considération pour une mesure précise et pertinente d'un point de vue fonctionnel, notamment pour une réutilisation lors de l'étude de la posture orthostatique.

Le Demandeur observe par ailleurs que les ergomètres existants utilisent généralement un capteur de forces ou de moments de force unidirectionnel : un tel capteur sous-estime les moments résultants à la cheville : en effet, une flexion plantaire (extension) et/ou une flexion dorsale (dorsiflexion) s'accompagnent toujours de mouvements d'inversion et d'adduction associés, dans les deux autres plans que le plan sagittal.

Les ergomètres existants ne permettent pas d'évaluer l'ensemble des contraintes exercées au niveau de l'articulation de la cheville.

L'utilisation d'un capteur unidirectionnel permet uniquement de connaître ce qui se passe autour de l'axe transversal de la cheville ; un tel capteur ne permet pas d'évaluer les moments accessoires à la flexion plantaire, c'est-à-dire ceux autour des axes sagittal (ou longitudinal) et vertical de la cheville (en inversion et en adduction).

Enfin, le Demandeur observe que les ergomètres existants sont souvent très encombrants et difficilement déplaçables ; par ailleurs, les ergomètres existants ne peuvent être utilisés que sur une seule jambe à la fois et ne peuvent pas être utilisés facilement en position alitée.

### Objet et Résumé de la présente invention

La présente invention, définie dans les revendications, vise à améliorer la situation décrite ci-dessus.

La présente invention vise à remédier aux différents inconvénients mentionnés ci-dessus en proposant un ergomètre, peu encombrant et peu coûteux, pouvant mesurer avec précision les forces exercées à l'articulation de la cheville par les muscles de flexion du pied d'un individu et exclusivement les forces exercées par ces muscles, et non celles exercées par d'autres muscles tels que les muscles extenseurs de hanche ou de genou.

A cet effet, l'objet de la présente invention concerne selon un premier aspect un ergomètre de type cheville pour mesurer les forces exercées sur l'articulation de la cheville d'un utilisateur par les muscles intervenant dans la mobilité de ladite cheville.

Selon la présente invention, l'ergomètre comporte une première partie et une deuxième partie, se présentant sous la forme d'une botte.

Avantageusement, la première partie est de type attelle ; elle est apte à recevoir un membre inférieur de l'utilisateur lorsque le genou de celui-ci est en extension.

Avantageusement, cette première partie comprend des moyens d'immobilisation qui sont configurés pour immobiliser le membre inférieur avec le genou en extension.

Avantageusement, la deuxième partie comprenant :
a) un corps principal fixé à la première partie,
b) un élément de contre-appui solidaire du corps principal,
c) une plaque rigide formant une surface d'appui pour la surface inférieure du pied lorsque le membre inférieur est immobilisé avec les moyens d'immobilisation, ladite plaque rigide étant sensiblement statique par rapport audit élément de contre-appui,
d) un capteur de force intercalé entre ladite plaque et ledit élément de contre-appui.

Selon l'invention, la plaque est non solidaire du corps principal de manière à ce que le capteur soit apte à mesurer la force exercée sur la surface d'appui de la plaque par les muscles intervenant dans la mobilité de ladite cheville, ladite force étant transmise de la plaque vers l'élément de contre-appui.

Par plaque statique par rapport à l'élément de contre-appui, il faut comprendre ici que la plaque ne bouge pas (d'un point de vue macroscopique) par rapport à l'élément de contre-appui (notamment par exemple lorsque l'utilisateur exerce une force avec son pied sur ladite plaque).

Par plaque non solidaire avec le corps principal, il faut comprendre ici que la plaque sur laquelle repose le pied de l'utilisateur est structurellement indépendante du corps principal, et donc de l'élément de contre-appui, le seul lien entre les deux éléments étant le capteur de force qui est intercalé entre la plaque et l'élément de contre-appui.

Ainsi, tant par sa structure que par sa configuration, l'ergomètre conçu dans le cadre de la présente invention se présente comme un appareil d'exercice simple dans son utilisation et précis dans la mesure effectuée.

L'agencement d'un capteur intercalé entre un élément de contre-appui et une plaque rigide, libre (non solidaire) et fixe (non statique) par rapport à l'élément de contre-appui permet d'avoir une mesure précise et fiable de la force exercée par les muscles de flexion du pied (ou muscles fléchisseurs plantaires) et transmise de la plaque vers l'élément de contre-appui.

Un tel capteur permet notamment une utilisation en « chaîne fermée ».

Il suffit de positionner au niveau de la première partie de l'ergomètre la jambe avec le genou en extension puis de l'immobiliser avec les moyens prévus à cet effet.

Dans cette position, le pied est en appui sur la plaque rigide.

Il suffit alors d'exercer une force de flexion (plantaire ou dorsale) sur la plaque.

Cette force va être transmise vers l'élément de contre-appui.

Le capteur de force, positionné entre la plaque et l'élément de contre-appui, peut ainsi mesurer cette force.

Pour mesurer la force exercée lors d'une flexion plantaire et/ou d'une flexion dorsale du pied de l'utilisateur, il est prévu avantageusement que le capteur de force soit du type capteur 3D (c'est-à-dire un capteur à trois dimensions).

De préférence, le capteur de force est un capteur de moment de force apte à mesurer les forces et les moments de force dans les trois dimensions (3D).

Selon l'invention, il est préféré une utilisation de l'ergomètre en « chaîne ouverte ».

A cet effet, l'ergomètre comporte en outre :
- une piste de roulement ou de glissement,
- un organe de roulement ou de glissement.

Cet organe de roulement ou de glissement est fixé au corps principal.

Dans un mode de réalisation avantageux, cet organe de roulement ou de glissement est positionné sensiblement au niveau du talon de la botte. Cet organe peut être positionné ailleurs si nécessaire, notamment pour des raisons techniques.

Avantageusement, l'organe de roulement ou de glissement est apte à coopérer avec la piste de roulement ou de glissement afin de limiter les frottements entre le corps principal et la piste.

La présence de cet organe sur le corps principal et la coopération de cet organe avec la piste rendent le dispositif indépendant de son environnement.

Cette configuration en « chaine ouverte » permet ainsi de mesurer avec une grande fiabilité et une grande précision la force exercée par les muscles de la cheville : cette configuration en « chaine ouverte » évite la mesure des compensations des muscles des autres articulations.

En effet, dans cette configuration, la présence de cet organe et de cette piste de roulement ou de glissement évite tout frottement susceptible de permettre un appui à l'utilisateur pour solliciter d'autres muscles tels que par exemple les muscles extenseurs de hanche.

Dans une variante, l'organe de roulement ou de glissement comprend une roulette pour un roulement du corps principal par rapport à la piste.

Une telle roulette permet un déplacement du corps par rapport à la piste avec un minimum de frottements.

Dans une variante alternative, l'organe de roulement ou de glissement comprend une surface de contact plane et la piste de roulement ou de glissement comprend une planche présentant une pluralité de billes réparties uniformément sur la planche.

Dans cette variante, l'organe de roulement ou de glissement peut rouler sur les billes de manière à se déplacer dans toutes les directions du plan de la planche avec un minimum de frottements.

Avantageusement, la plaque comprend au niveau de ses bords latéraux des éléments de rigidification ; ceux-ci s'étendent perpendiculairement par rapport à la plaque.

Ces éléments de rigidification réduisent, voire suppriment, les éventuelles déformations de la plaque, lors par exemple d'une flexion du pied.

De préférence, les moyens d'immobilisation comprennent un jeu de sangles pour maintenir immobilisé le membre inférieur de l'utilisateur.

De préférence, la deuxième partie comprend un chausson pour recevoir le pied de l'utilisateur, ledit chausson étant fixé sur la plaque de manière à maintenir solidaire le pied avec la plaque.

Grâce à un tel chausson, le pied est solidaire de la plaque.

Alternativement ou de façon complémentaire, on peut également prévoir un jeu de sangles pour le maintien du pied.

Avantageusement, l'ergomètre selon la présente invention comporte des premiers moyens de réglage, dit angulaire, qui sont configurés pour régler la position angulaire de la deuxième partie par rapport à la première partie.

De tels moyens de réglage permettent de régler l'inclinaison du pied sur la surface d'appui de la plaque.

Un tel réglage permet d'évaluer la relation entre l'inclinaison de la cheville et les forces développées.

Dans un mode de réalisation particulier, les moyens de réglage comprennent :
- une paire d'écrous rétractables positionnés sur la première partie, et
- une pluralité de trous de réglage ménagés sur le corps principal de la deuxième partie.

Dans ce mode, les écrous sont aptes à être introduits dans les trous de réglage pour le réglage et le verrouillage de la position angulaire de la deuxième partie par rapport à la première partie.

L'homme du métier comprendra ici que les moyens de réglage ci-dessus peuvent être de façon plus générale un système de verrouillage à ressorts apte à coopérer avec des rainures ménagées sur le corps principal.

Avantageusement, l'ergomètre selon la présente invention comporte des deuxièmes moyens de réglage, dit en hauteur, qui sont configurés pour régler la hauteur de la première partie par rapport à la deuxième partie.

Un tel réglage en hauteur permet d'ajuster l'ergomètre en fonction de la taille de l'utilisateur, et notamment la taille de la jambe de l'utilisateur.

Avantageusement, l'ergomètre selon la présente invention comporte une unité informatique de traitement qui est apte à recevoir et traiter les signaux de mesure provenant du capteur afin de déterminer la force exercée par les muscles intervenant dans les mouvements de la cheville.

L'utilisation d'un ergomètre tel que décrit ci-dessus permet de réaliser des exercices de rééducation prédéterminés et adaptés à l'utilisateur.

L'objet de la présente invention concerne selon un deuxième aspect l'utilisation d'un ergomètre tel que décrit ci-dessus pour réaliser des tests de force sur une personne afin d'évaluer la capacité de cette personne à maintenir une posture orthostatique, notamment pour autoriser une personne alitée à reprendre la station debout et/ou une activité.

Ainsi, l'objet de la présente invention, par ses différents aspects fonctionnels et structurels décrits ci-dessus, permet une mesure précise et fiable de la force produite par les muscles fléchisseurs et/ou extenseurs de la cheville, en limitant l'influence des forces exercées par d'autres muscles, comme c'est le cas dans les autres ergomètres développés jusqu'à présent.

### Brève description des figures annexées

D'autres caractéristiques et avantages de la présente invention ressortiront de la description ci-dessous, en référence aux figures 4 à 6 annexées qui en illustrent un mode de réalisation dépourvu de tout caractère limitatif et sur lesquelles :
- la figure 4 représente de façon schématique une vue latérale d'un ergomètre selon un exemple de réalisation ;
- la figure 5 représente de façon schématique une vue frontale d'un ergomètre conforme à la figure 4 ;
- la figure 6 représente de façon schématique une vue latérale d'un ergomètre conforme à la figure 4 avec un organe et une piste de roulement ou de glissement.

### Description détaillée de différents exemples de mise en œuvre

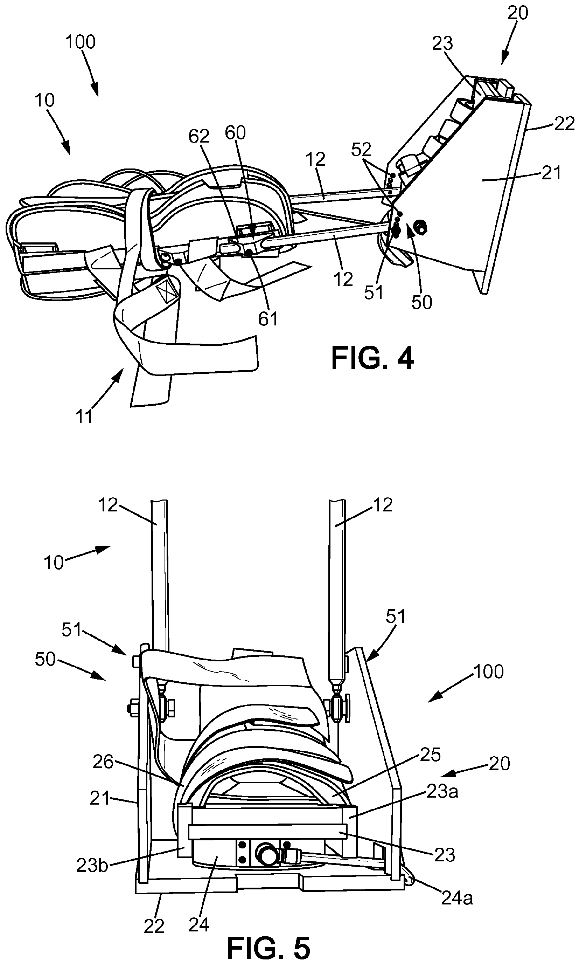

Un ergomètre selon un exemple de réalisation de la présente invention va maintenant être décrit dans ce qui va suivre en faisant référence conjointement aux figures 4 à 6.

Pour mémoire, lors d'une contraction isométrique des muscles de la cheville vers l'extension, on remarque sur la plupart des ergomètres de l'état de la technique que l'utilisateur se sert de l'appui de son dos ou de ses épaules pour pousser sur la pédale de l'ergomètre, ce qui implique une participation d'autres muscles sur d'autres articulations induisant une surestimation de l'évaluation des forces (ou des moments de force) développées sur l'articulation de la cheville.

Il est également observé que les ergomètres du type cheville qui positionnent l'utilisateur en position assise, avec le genou fléchi à 90° pour limiter la participation de l'articulation du genou, réduisent les efforts des muscles gastrocnémiens.

Or, il est important d'évaluer les forces développées par ces muscles gastrocnémiens sur la cheville.

Il est donc préférable de réaliser les tests avec le genou en extension pour tenir compte de ces forces.

Un des objectifs de la présente invention est donc de concevoir un ergomètre apte à mesurer, en position jambe tendue (c'est-à-dire genou en extension), la force maximale développée par les muscles fléchisseurs et/ou par les muscles extenseurs de cheville ; c'est-à-dire les muscles permettant la stabilisation de la cheville dans le plan sagittal (flexion plantaire et dorsiflexion).

Ceci est rendu possible dans l'exemple qui va suivre avec un ergomètre 100 de type cheville tel que celui illustré en figure 4.

Dans l'exemple décrit ici, l'ergomètre 100 comprend une première partie 10 du type attelle.

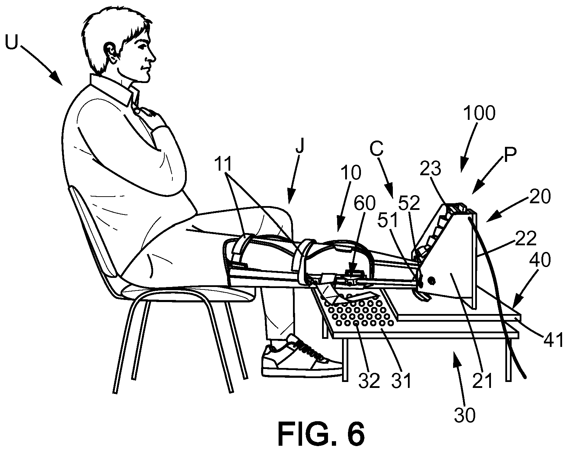

Dans cet exemple, cette première partie 10 est une attelle de Zimmer : ladite attelle est apte à recevoir le membre inférieur de l'utilisateur U lorsque le genou de l'utilisateur est en extension comme illustré en figure 6.

Bien évidemment, l'homme du métier comprendra ici que d'autres types d'attelle peuvent être utilisés comme par exemple une attelle en forme de gouttière recevant la cuisse et la jambe. D'autres types d'attelle peuvent encore être envisagés.

Cette attelle est composée de deux tiges 12 rigides et longilignes qui s'étendent le long de la jambe de l'utilisateur U.

Ces tiges 12 sont ajustables et comprennent des moyens de réglage 60 en hauteur pour régler la hauteur de l'attelle en fonction de la taille de l'utilisateur.

Dans cet exemple et comme illustré en figure 4, ces moyens de réglage 60 consistent en un mécanisme de glissière 62 permettant la coulissement des tubes 12 pour régler la hauteur et un jeu de vis 61 permettant de bloquer en position les tubes, une fois la hauteur ajustée.

Il suffit donc à l'utilisateur U de positionner sa jambe dans l'attelle après avoir réglé préalablement la hauteur de l'attelle si nécessaire.

Comme illustré en figure 6, l'attelle comprend des moyens d'immobilisation 11, par exemple un jeu de sangles, pour immobiliser le membre inférieur de l'utilisateur U lorsque le genou est en extension.

L'homme du métier comprendra ici qu'il est possible d'envisager d'autres moyens que les sangles pour immobiliser le membre inférieur.

Une fois en position, l'utilisateur U avec le genou en extension peut serrer les sangles pour immobiliser son membre inférieur.

Dans l'exemple décrit ici, l'ergomètre 100 comprend une deuxième partie 20, appelée également plateforme de force 3D.

Dans cet exemple, cette deuxième partie 20 comprend un corps principal 21 fixé à la première partie 10. Ce corps principal 21 englobe le pied de l'utilisateur U.

Comme illustré en figure 5, cette deuxième partie 20 comprend un élément de contre-appui 22.

Cet élément de contre-appui 22 consiste en une plaque solidaire du corps principal 21.

L'homme du métier comprendra ici que l'élément de contre-appui 22 et le corps principal 21 peuvent constituer une seule et même pièce monobloc, obtenue par exemple par moulage.

Dans l'exemple décrit ici, et comme illustré en figure 5, la deuxième partie 20 comprend également une autre plaque rigide 23 qui forme une surface d'appui pour la surface inférieure du pied de l'utilisateur U lorsque le membre inférieur est immobilisé par l'attelle avec les moyens d'immobilisation 11.

Dans l'exemple décrit ici et illustré en figure 5, cette plaque 23 est sensiblement parallèle à l'élément de contre-appui 22.

Dans cet exemple, la plaque 23 est fixe par rapport à l'élément de contre-appui 22.

Ainsi, cette plaque 23 est statique ; c'est-à-dire qu'elle ne bouge pas et ne se déforme pas, d'un point de vue macroscopique, par rapport à l'élément de contre-appui 22, même lorsque le pied de l'utilisateur exerce une force sur ladite plaque 23.

Afin de limiter davantage toute déformation, la plaque 23 présente sur ses bords latéraux des éléments de rigidification 23a et 23b (voir figure 5).

Entre cette plaque 23 et l'élément de contre-appui 22, un capteur de force 24 est intercalé.

L'agencement d'un tel capteur de force 24 intercalé entre l'élément de contre-appui 22 et la plaque 23 est caractéristique de la présente invention.

En effet, lorsque l'utilisateur U exerce une force sur la plaque 23 par l'intermédiaire de son pied, cette force est transmise de la plaque 23 vers l'élément de contre-appui 22.

Le capteur de force 24 positionné entre les deux éléments 22 et 23 peut alors mesurer cette force transmise.

Dans cet exemple, le capteur 24 est un capteur de force 3D qui contient un capteur de force-couple du type SH100D1002A2-2 Sensix.

Un tel capteur de force 24 permet de mesurer les forces lors d'une flexion plantaire et lors d'une flexion dorsale.

Il est prévu que le pied de l'utilisateur U soit logé dans un chausson 25 fixé solidairement sur la surface d'appui de la plaque 23.

De façon complémentaire, il est également possible de prévoir un jeu de sangles 26 tel que par exemple :
- deux bandes de fixation auto-adhésives repositionnables pour maintenir en position le pied de l'utilisateur U,
- d'autres bandes de fixation auto-adhésives repositionnables complémentaires fixées sur la plateforme de force pour bloquer le mouvement de la cheville, l'une passant à l'arrière du talon, l'autre passant sur le devant de l'articulation de la cheville.

Il est encore possible de prévoir une autre bande de fixation auto-adhésive repositionnable placée au niveau de la face dorsale de l'articulation métatarso-phalangienne afin de bloquer tout mouvement de l'avant-pied.

Une fois l'ensemble de ces éléments d'immobilisation positionnés, le pied de l'utilisateur U est solidaire de la surface d'appui de la plaque 23 : dans cette configuration, le pied de l'utilisateur U est fermement maintenu en place par un chausson fixé 25 à la plaque 23, cette plaque 23 étant elle-même fixée sur la plateforme de force.

Comme illustré en figure 6, l'ergomètre 100 selon la présente invention s'utilise donc en position assise (ou en position allongée), le membre inférieur de l'utilisateur U étant placé dans l'attelle avec le genou en extension et le pied fixé dans le chausson 25 étant attaché solidairement à la plateforme de l'ergomètre 100.

De préférence, l'axe de rotation de la cheville est aligné avec l'axe de l'ergomètre.

La jambe est en extension au niveau de l'articulation du genou et l'articulation de la cheville est en position neutre : l'axe longitudinal du pied forme un angle de 90° avec celui de la jambe dans le plan sagittal, comme il peut l'être lors du maintien de la position orthostatique.

Il est toutefois possible de régler l'inclinaison angulaire de l'articulation de la cheville.

Ceci est rendu possible grâce à des moyens de réglage angulaire 50 qui permettent de régler l'angle formé par l'axe du pied P et celui de la jambe J.

Comme illustré en figures 4 et 5, ces moyens de réglage 50 consistent en un jeu d'écrous 51 rétractables positionnés au niveau de la portion inférieure des tubes 12, les tubes étant fixés au corps principal 21 par un pivot.

Le corps principal 21 présentant des trous de réglage 52 pour l'introduction desdits écrous 51.

En choisissant le trou de réglage 52, il est possible pour l'utilisateur U de régler l'inclinaison de son pied par rapport à l'axe de sa jambe.

Dans l'exemple décrit ici, on utilise l'ergomètre en « chaîne ouverte ».

Selon cette configuration, le talon de la botte (c'est-à-dire le talon de la deuxième partie) comprend un organe de roulement 40.

Dans cet exemple, l'organe 40 est constitué par une planche 41 fixée directement ou indirectement au talon de la botte (c'est-à-dire à l'arrière du corps principal 21).

Cette planche 41 est elle-même placée sur une piste de roulement 50 formée ici par une autre planche 52.

Dans cet exemple, cette planche 52 présente sur sa surface de contact avec la planche 41 des billes de roulement 51.

Le déplacement relatif de la planche 41 sur les billes 51 permet de rendre négligeable tout frottement entre l'ergomètre 100 et le support.

On comprendra ici que d'autres moyens sont possibles pour éviter de tels frottements, comme par exemple la présence d'une roulette sur le talon de la botte.

Dans l'exemple décrit, il est prévu pour effectuer les acquisitions un câble 24a reliant le capteur de force 24 de l'ergomètre à une unité informatique de traitement (non représentée ici).

Les signaux d'efforts mesurés par le capteur 24 sont alors envoyés par ce câble 24a vers l'unité de traitement.

Alternativement, on peut prévoir que ces signaux soient envoyés par des moyens de communication sans fil.

Ces signaux sont ensuite directement enregistrés par un logiciel de traitement du type « *Labview Signal Express* » ou « *Matlab* »*,* avec par exemple une fréquence d'échantillonnage de 100 Hz.

Dans l'exemple décrit ici, cette unité de traitement comporte un processeur qui peut être configuré pour réaliser des tests de force maximaux isométriques ou faire des exercices adaptés de rééducation.

Par exemple, un tel processeur peut être configuré pour demander à l'utilisateur de réaliser des contractions explosives (montées en force rapide) puis des contractions réalisées progressivement sur 5 secondes jusqu'à intensité maximale (condition de rampe : ici on peut par exemple utiliser un métronome battant à 60 bpm pour que le sujet réalise une montée en force régulière sur 5 secondes).

L'unité de traitement peut être reliée à un module d'affichage (non représenté ici) pour une représentation de ces objectifs à atteindre et une visualisation en temps réel des efforts fournis par les muscles, par exemple sous la forme d'un graphique représentant en temps réel la force mesurée et l'objectif à atteindre.

Ce module d'affichage permet ainsi à l'utilisateur de maîtriser les efforts à fournir par exemple lorsqu'il s'agit d'exercice où des efforts cibles déterminés au préalable doivent être atteints.

L'ergomètre 100 selon la présente invention se présente aussi comme un appareil d'évaluation d'objectif permettant, grâce à la présence d'un capteur 24 de force 3D intercalé entre une plaque d'appui 23 qui est libre et une plaque 22 servant de contre-appui, la mesure précise et fiable des efforts exercés sur l'articulation de la cheville par les muscles péri-articulaires.

Un tel ergomètre 100 se présente ainsi comme un appareil de mesure fiable, précis et abordable sur le plan financier ; il est par ailleurs facile à transporter, ce qui offre la possibilité d'amener l'instrument de mesure à l'utilisateur (par exemple jusqu'à son lit), et non l'inverse comme c'est généralement le cas avec les ergomètres existants.

Il peut donc se transporter facilement, ce qui est très appréciable si l'on souhaite par exemple tester des patients, et notamment des personnes âgées ou des personnes souffrant d'instabilité chronique de la cheville, qui restent difficilement déplaçables.

De la même façon, il est possible de le transporter dans les centres ou les salles ou les terrains de sports pour des sportifs afin d'être utilisé lors des entraînements ou des préparations physiques.

Par ailleurs, en plus des avantages ci-dessus, il est possible de réaliser les mesures simultanément sur les deux membres inférieurs en utilisant deux ergomètres. On parle de test bilatéral.

On sait en effet que la somme des forces maximales exercées simultanément par chacun des muscles des deux membres inférieurs lors d'une contraction bilatérale est plus faible que la somme des forces maximales exercées lors de contractions unilatérales réalisées par ces mêmes muscles.

Ceci est connu sous le terme de déficit bilatéral.

L'utilisation simultanée de deux ergomètres 100 selon l'invention pour un test bilatéral met en évidence ce déficit bilatéral, lorsque les ergomètres sont utilisés en configuration « chaîne fermée » ; c'est-à-dire en prenant appui contre une butée.

Cependant, en configuration « chaîne ouverte », on ne retrouve pas un tel déficit bilatéral.

La configuration « chaîne fermée » permet, lors des contractions unilatérales d'utiliser les ajustements posturaux pour pousser plus fort, ce qui n'est pas possible en configuration « chaîne ouverte ».

Lors d'une poussée bilatérale en « chaîne ouverte », les ajustements posturaux ne sont pas possibles et la force mesurée pour chaque ergomètre 100 correspond uniquement à la force développée par les muscles fléchisseurs plantaires ou fléchisseurs dorsaux de la cheville.

Ainsi, ces tests en bilatéral avec deux ergomètres 100 utilisés en simultané permettent de montrer qu'il n'existe pas réellement de déficit bilatéral, mais qu'il existe plutôt une facilitation unilatérale en chaîne fermée. En d'autres termes, que ce soit en condition unilatérale ou bilatérale, la force développée au niveau de chaque cheville reste identique, ce qui confirme l'absence d'un déficit bilatéral.

Il devra être observé que cette description détaillée porte sur un exemple de réalisation particulier de la présente invention, mais qu'en aucun cas cette description ne revêt un quelconque caractère limitatif à l'objet de l'invention ; bien au contraire, elle a pour objectif d'ôter toute éventuelle imprécision ou toute mauvaise interprétation des revendications qui suivent.

## Revendications

1. Ergomètre (100) de type cheville pour mesurer une force exercée sur l'articulation de la cheville (C) d'un membre inférieur d'un utilisateur (U) par les muscles intervenant dans la mobilité de ladite cheville (C),
ledit ergomètre (100), se présentant sous la forme d'une botte, comportant :
- une première partie (10) de type attelle apte à recevoir ledit membre inférieur lorsque le genou dudit utilisateur (U) est en extension, et comprenant des moyens d'immobilisation (11) configurés pour immobiliser ledit membre inférieur avec le genou en extension, et
- une deuxième partie (20) comprenant :
a) un corps principal (21) fixé à la première partie (10),
b) un élément de contre-appui (22) solidaire dudit corps principal (21),
c) une plaque rigide (23) formant une surface d'appui pour la surface inférieure dudit pied lorsque ledit membre inférieur est immobilisé avec les moyens d'immobilisation (11), ladite plaque rigide (23) étant sensiblement statique par rapport audit élément de contre-appui (22),
d) un capteur de force (24) intercalé entre ladite plaque (23) et ledit élément de contre-appui (22),
- ladite plaque (23) étant non solidaire dudit corps principal (21) de manière à ce que ledit capteur (24) est apte à mesurer la force exercée sur la surface d'appui de la plaque (23) par les muscles intervenant dans la mobilité de ladite cheville (C), ladite force étant transmise de la plaque (23) vers l'élément de contre-appui (22),
et dans lequel le ergomètre comporte :
- une piste de roulement ou de glissement (30),
- un organe de roulement ou de glissement (40) fixé directement ou indirectement au corps principal (21), ledit organe (40) étant apte à coopérer avec ladite piste (30) afin de limiter les frottements entre ledit corps principal (21) et ladite piste (30) et de sorte ledit ergomètre est configuré pour mesurer avec précision les forces exercées à l'articulation de la cheville par les muscles de flexion du pied d'un individu et exclusivement les forces exercées par ces muscles de flexion, et non les forces exercées par les muscles extenseurs de hanche ou de genou.

2. Ergomètre (100) selon la revendication 1, dans lequel le capteur de force (24) est du type capteur 3D pour mesurer une force exercée lors d'une flexion plantaire et/ou d'une flexion dorsale du pied de l'utilisateur (U).

3. Ergomètre (100) selon la revendication 2, dans lequel ledit capteur de force (24) est un capteur de moments de forces, apte à mesurer les forces et les moments de force dans les trois dimensions.

4. Ergomètre (100) selon la revendication 3, dans lequel ledit organe (40) comprend une roulette pour un roulement du corps principal (21) par rapport à ladite piste (30).

5. Ergomètre (100) selon la revendication 3, dans lequel ledit organe (40) comprend une surface de contact plane (41) et dans lequel ladite piste (30) comprend une planche (31) présentant une pluralité de billes (32) réparties uniformément sur ladite planche (31).

6. Ergomètre (100) selon l'une des revendications 1 à 5, dans lequel ladite plaque (23) comprend au niveau de ses bords latéraux des éléments de rigidification (23a, 23b) qui s'étendent perpendiculairement par rapport à ladite plaque (23).

7. Ergomètre (100) selon l'une quelconque des revendications 1 à 6, dans lequel les moyens d'immobilisation (11) comprennent un jeu de sangles pour maintenir immobilisé le membre inférieur de l'utilisateur (U).

8. Ergomètre (100) selon l'une quelconque des revendications 1 à 7, dans lequel la deuxième partie (20) comprend un chausson (25) pour recevoir le pied de l'utilisateur (U), ledit chausson (25) étant fixé sur la plaque (23) de manière à maintenir solidaire ledit pied de l'utilisateur (U) avec ladite plaque (23).

9. Ergomètre (100) selon l'une quelconque des revendications 1 à 8, comportant des premiers moyens de réglage (50), dit angulaire, configurés pour régler la position angulaire de la deuxième partie (20) par rapport à la première partie (10).

10. Ergomètre (100) selon la revendication 9, dans lequel les moyens de réglage angulaire (50) comprennent :
- une paire d'écrous (51) rétractables positionnés sur la première partie (10), et
- une pluralité de trous de réglage (52) ménagés sur le corps principal (21) de la deuxième partie (20),
lesdits écrous (51) étant aptes à être introduits dans les trous de réglage (52) pour le réglage et le verrouillage de la position angulaire de la deuxième partie (20) par rapport à la première partie (10).

11. Ergomètre (100) selon l'une quelconque des revendications 1 à 10, comportant des deuxièmes moyens de réglage (60), dit en hauteur, configurés pour régler la hauteur de la premier partie (10) par rapport à la deuxième partie (20).

12. Ergomètre (100) selon l'une quelconque des revendications 1 à 11, comportant une unité informatique de traitement apte à recevoir et traiter les signaux de mesure provenant dudit capteur (24) afin de déterminer la force exercée par les muscles intervenant dans les mouvements de la cheville.

13. Utilisation d'un ergomètre (100) selon l'une des revendications 1 à 12 pour réaliser des tests de force sur un patient afin d'évaluer la capacité d'une personne à maintenir une posture orthostatique, notamment pour autoriser une personne à reprendre la station debout et/ou une activité.

## Patentansprüche

1. Ergometer (100) vom Fußgelenktyp zum Messen einer Kraft, die auf das Fußgelenk (C) einer unteren Gliedmaße eines Benutzers (U) durch die Muskeln ausgeübt wird, die an der Bewegung des Fußgelenks (C) beteiligt sind,
wobei das Ergometer (100), das in Form eines Stiefels vorliegt, umfasst:
- einen ersten Teil (10) vom Typ Schiene, der die untere Gliedmaße aufnehmen kann, wenn das Knie des Benutzers (U) gestreckt ist, und umfassend Immobilisierungsmittel (11), die dazu ausgebildet sind, die untere Gliedmaße mit dem gestreckten Knie zu immobilisieren, und
- einen zweiten Teil (20), der Folgendes umfasst:
a) einen Hauptkörper (21), der an dem ersten Teil (10) befestigt ist,
b) ein Gegenstützelement (22), das fest mit dem Hauptkörper (21) verbunden ist,
c) eine starre Platte (23), die eine Auflagefläche für die untere Fläche des Fußes bildet, wenn die untere Gliedmaße mit den Immobilisierungsmitteln (11) immobilisiert ist, wobei die starre Platte (23) in Bezug auf das Gegenstützelement (22) im Wesentlichen statisch ist,
d) einen Kraftsensor (24), der zwischen der Platte (23) und dem Gegenstützelement (22) angeordnet ist,
- wobei die Platte (23) nicht fest mit dem Hauptkörper (21) verbunden ist, so dass der Sensor (24) dazu in der Lage ist, die Kraft zu messen, die auf die Auflagefläche der Platte (23) durch diejenigen Muskeln ausgeübt wird, die an der Beweglichkeit des Fußgelenks (C) beteiligt sind, wobei die Kraft von der Platte (23) zum Gegenstützelement (22) übertragen wird,
und wobei das Ergometer umfasst:
- eine Roll- oder Gleitbahn (30),
- ein Roll- und Gleitelement (40), das direkt oder indirekt am Hauptkörper (21) befestigt ist, wobei das Element (40) dazu in der Lage ist, mit der Bahn zusammenzuwirken, um Reibungen zwischen dem Hauptkörper (21) und der Bahn (30) zu begrenzen und der Ergometer ist derart ausgebildet, dass er die von den Fußbeugemuskeln einer Person auf das Fußgelenk ausgeübte Kraft präzise messen kann, und zwar ausschließlich die von diesen Beugemuskeln ausgeübten Kräfte und nicht die von den Hüft- oder Kniestreckermuskeln ausgeübten Kräfte.

2. Ergometer (100) nach Anspruch 1, wobei der Kraftsensor (24) vom Typ 3D-Sensor ist, um eine Kraft zu messen, die bei einer Plantarflexion und/oder einer Dorsalflexion des Fußes des Benutzers (U) ausgeübt wird.

3. Ergometer (100) nach Anspruch 2, wobei der Kraftsensor (24) ein Kraftmomentsensor ist, der in der Lage ist, Kräfte und Kraftmomente in drei Dimensionen zu messen.

4. Ergometer (100) nach Anspruch 3, wobei das Element (40) eine Rolle für ein Abrollen des Hauptkörpers (21) relativ zu der Bahn (30) umfasst.

5. Ergometer (100) nach Anspruch 3, wobei das Element (40) eine ebene Kontaktfläche (41) umfasst und wobei die Bahn (30) ein Brett (31) umfasst, das eine Vielzahl von Kugeln (32) aufweist, die gleichmäßig über das Brett (31) verteilt sind.

6. Ergometer (100) nach einem der Ansprüche 1 bis 5, wobei die Platte (23) an ihren Seitenkanten Versteifungselemente (23a, 23b) aufweist, die sich senkrecht zu der Platte (23) erstrecken.

7. Ergometer (100) nach einem der Ansprüche 1 bis 6, wobei die Immobilisierungsmittel (11) einen Satz von Gurten umfassen, um die untere Extremität des Benutzers (U) immobilisiert zu halten.

8. Ergometer (100) nach einem der Ansprüche 1 bis 7, wobei der zweite Teil (20) einen Innenschuh (25) zur Aufnahme des Fußes des Benutzers (U) umfasst, wobei der Innenschuh (25) an der Platte (23) derart befestigt ist, dass der Fuß des Benutzers (U) fest mit der Platte (23) verbunden bleibt.

9. Ergometer (100) nach einem der Ansprüche 1 bis 8, mit ersten Einstellmitteln (50), sogenannten Winkeleinstellmitteln, die dazu ausgebildet sind, die Winkelposition des zweiten Teils (20) in Bezug auf den ersten Teil (10) einzustellen.

10. Ergometer (100) nach Anspruch 9, wobei die Mittel zur Winkeleinstellung (50) Folgendes umfassen:
- ein Paar zurückziehbare Muttern (51), die auf dem ersten Teil (10) positioniert sind, und
- eine Vielzahl von Einstelllöchern (52), die auf dem Hauptkörper (21) des zweiten Teils (20) bereitgestellt sind,
wobei die Muttern (51) dazu geeignet sind, in die Einstelllöcher (52) eingesetzt zu werden, um die Winkelposition des zweiten Teils (20) relativ zum ersten Teil (10) einzustellen und zu sichern.

11. Ergometer (100) nach einem der Ansprüche 1 bis 10, mit zweiten Einstellmitteln (60), sogenannten Höheneinstellmitteln, die dazu ausgebildet sind, die Höhe des ersten Teils (10) in Bezug auf den zweiten Teil (20) einzustellen.

12. Ergometer (100) nach einem der Ansprüche 1 bis 11, mit einer Datenverarbeitungseinheit, die in der Lage ist, die von dem Sensor (24) stammenden Messsignale zu empfangen und zu verarbeiten, um die Kraft zu bestimmen, die von den Muskeln ausgeübt wird, die an den Bewegungen des Fußgelenks beteiligt sind.

13. Verwendung eines Ergometers (100) nach einem der Ansprüche 1 bis 12 zur Durchführung von Krafttests an einem Patienten, um die Fähigkeit einer Person zur Aufrechterhaltung einer orthostatischen Körperhaltung zu bewerten, insbesondere um einer Person zu erlauben, wieder zu stehen und/oder eine Aktivität aufzunehmen.

## Claims

1. An ankle-type ergometer (100) for measuring a force exerted on the ankle joint (C) of a lower limb of a user (U) by the muscles involved in the mobility of said ankle (C), said ergometer (100), in the form of a boot, including:
- a first portion (10) of the splint type capable of receiving said lower limb when the knee of said user (U) is extended, and comprising immobilisation means (11) configured to immobilise said lower limb with the extended knee, and
- a second portion (20) comprising:
a) a main body (21) fixed to the first portion (10),
b) a counter-bearing element (22) secured to said main body (21),
c) a rigid plate (23) forming a bearing surface for the lower surface of said foot, when said lower limb is immobilised with the immobilisation means (11), said rigid plate (23) being substantially static relative to said counter-bearing element (22),
d) a force sensor (24) interposed between said plate (23) and said counter-bearing element (22).
- said plate (23) being not secured to said main body (21) so that said sensor (24) is capable of measuring the force exerted on the bearing surface of the plate (23) by the muscles involved in the mobility of said ankle (C), said force being transmitted from the plate (23) to the counter-bearing element (22).
and wherein the ergometer includes:
- a rolling or sliding track (30),
- a rolling or sliding member (40) directly or indirectly fixed to the main body (21), said member (40) being capable of cooperating with said track (30) in order to limit friction between said main body (21) and said track (30) and so said ergometer is configured to accurately measure the forces exerted at the ankle joint by the flexor muscles of the foot of an individual and exclusively the forces exerted by these flexor muscles, and not the forces exerted by the hip or knee extensor muscles.

2. The ergometer (100) according to claim 1, wherein the force sensor (24) is of the 3D sensor type for measuring a force exerted during plantar flexion and/or dorsal flexion of the user's foot (U).

3. The ergometer (100) according to claim 2, wherein said force sensor (24) is a force moment sensor, capable of measuring the forces and moments of force in three dimensions.

4. The ergometer (100) according to claim 3, wherein said member (40) comprises a roller for rolling the main body (21) relative to said track (30).

5. The ergometer (100) according to claim 3, wherein said member (40) comprises a planar contact surface (41) and wherein said track (30) comprises a board (31) having a plurality of balls (32) which are evenly distributed on said board (31).

6. The ergometer (100) according to one of claims 1 to 5, wherein said plate (23) comprises, at the lateral edges thereof, stiffening elements (23a, 23b) which extend perpendicularly relative to said plate (23).

7. The ergometer (100) according to any one of claims 1 to 6, wherein the immobilisation means (11) comprise a set of straps to hold the lower limb of the user (U) stationary.

8. The ergometer (100) according to any one of claims 1 to 7, wherein the second portion (20) comprises a slipper (25) for receiving the foot of the user (U), said slipper (25) being fixed on the plate (23) so as to hold said user's foot (U) secured to said plate (23).

9. The ergometer (100) according to any one of claims 1 to 8, including adjustment means (50), called angular adjustment means, configured to adjust the angular position of the second portion (20) relative to the first portion (10).

10. The ergometer (100) according to claim 9, wherein the angular adjustment means (50) comprise:
- a pair of retractable nuts (51) positioned on the first portion (10), and
- a plurality of adjustment holes (52) formed on the main body (21) of the second portion (20).
said nuts (51) being capable of being inserted into the adjustment holes (52) for adjusting and locking the angular position of the second portion (20) relative to the first portion (10).

11. The ergometer (100) according to any one of claims 1 to 10, including second adjustment means (60), called height adjustment means, configured to adjust the height of the first portion (10) relative to the second portion (20).

12. The ergometer (100) according to any one of claims 1 to 11, including a computer processing unit capable of receiving and processing the measurement signals from said sensor (24) in order to determine the force exerted by the muscles involved in the movements of the ankle.

13. Use of an ergometer (100) according to one of claims 1 to 12, to carry out force tests on a patient in order to assess the ability of a person to maintain an orthostatic posture, in particular to enable a person to resume the standing position or an activity.
